# EUROPEAN PATENT SPECIFICATION

(11) **EP 2 851 003 B1**
(45) Date of publication and mention of the grant of the patent: **05.10.2016**
(21) Application number: 13791179.8
(22) Date of filing: 10.05.2013
(51) Int. Cl.: A61B 5/151

(54) **INJECTOR**
INJEKTOR
INJECTEUR

(30) Priority: 18.05.2012 JP 2012114721
(43) Date of publication of application: 25.03.2015
(73) Proprietor: Asahi Polyslider Co., Ltd., Osaka-shi, Osaka 530-0005 (JP)
(72) Inventor: SAEKI, Hideaki, Maniwa-shi Okayama 719-3226 (JP); IMORI, Hirokazu, Maniwa-shi Okayama 719-3226 (JP)
(74) Representative: Hoffmann Eitle
(86) International application number: PCT/JP2013/063181
(87) International publication number: WO 2013/172268

(56) References cited:
- EP-A1- 1 405 595
- WO-A1-2008/105373
- WO-A2-01/28423
- WO-A2-2006/107914
- DE-A1-102009 059 035
- JP-A- 2010 035 687
- JP-A- 2012 075 621

## Description

### TECHNICAL FIELD

The present invention relates to an injector used as a pricking device. More particularly, the present invention relates to an injector capable of launching "blood-sampling lancet" so as to provide a pricking.

### BACKGROUND OF THE INVENTION

In order to measure a blood sugar level of the patient with diabetes, it is required to take a sample of the blood from the patient. Various kinds of pricking devices have been used for sampling a small amount of the blood. The pricking device is generally composed of a lancet (see Patent Document listed below) and an injector. The lancet serves to actually prick. While on the other hand, the injector has a function of launching the lancet toward a predetermined region.

Specifically, the lancet has a pricking needle, whereas the injector has a plunger equipped with a lancet-attachment portion and a spring. The spring of the plunger is used in its compressed state. The releasing of the compressed spring can give a driving force for launching the plunger. In use of the pricking device, the lancet is attached to the plunger of the injector, and thereafter the compressed state of the spring is released by actuating of a trigger. Due to an expansion of the compressed spring, the plunger with the lancet attached thereto is launched toward the predetermined region so that the predetermined region is pricked by the lancet.

### PATENT DOCUMENTS (RELATED ART PATENT DOCUMENTS)

PATENT DOCUMENT 1: U.S. Patent Publication No. 5,385,571 DE 10 2009 059 035 A1 discloses features falling under the preamble of claim 1. WO 2006/107914 A2 is further prior art.

### DISCLOSURE OF THE INVENTION

### PROBLEMS TO BE SOLVED BY THE INVENTION

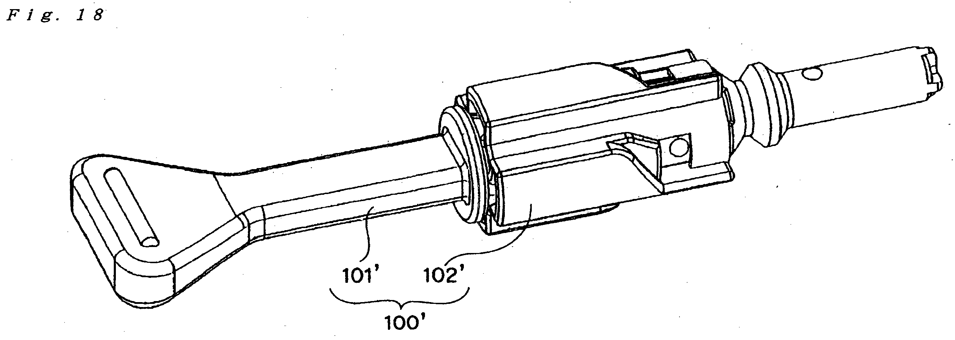

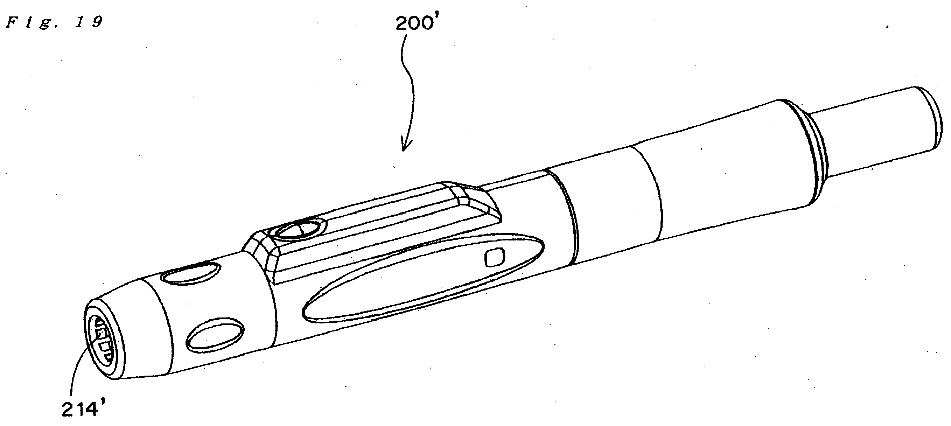

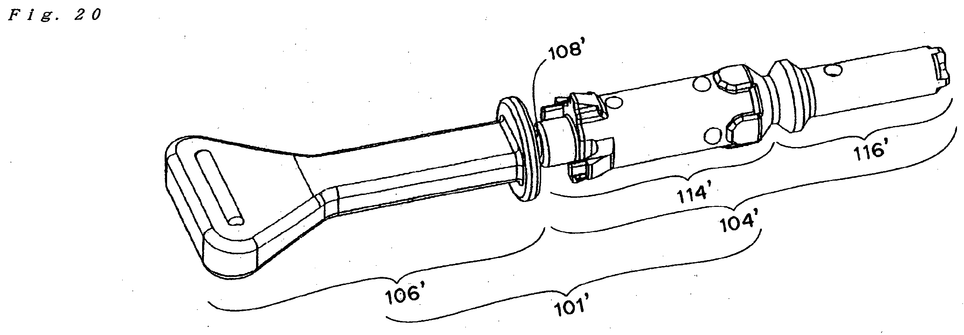

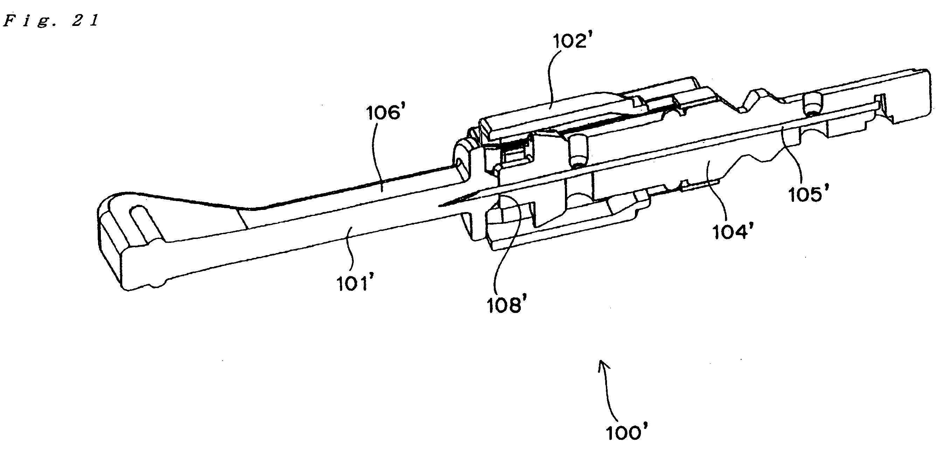

The present applicant has invented the following pricking device, and filed the application regarding such device (WO 2007/018215 A1, filed date: 8 Aug 2006, title of the invention: "PRICKING DEVICE, AS WELL AS LANCET ASSEMBLY AND INJECTOR ASSEMBLY THAT CONSTITUTE THE PRICKING DEVICE"). Referring to the accompanying drawings, the lancet assembly and the injector assembly invented by the applicant will be briefly explained below (note that the term "*injector assembly*" will be hereinafter referred to also as "*injector*"). Fig. 18 shows an external appearance of a lancet assembly 100' , and Fig. 19 shows an external appearance of an injector 200'. As shown in Fig. 18, the lancet assembly 100' is composed of a lancet 101' and a protective cover 102'. As shown in Figs. 20 and 21, the lancet 101' is composed of a lancet body 104', lancet cap 106' and a pricking component 105' . The pricking component 105' is situated in both of the lancet body 104' and the lancet cap 106' , pricking component 105' being made of metal, the lancet body 104' and the lancet cap 106' being made of resin. The tip of the pricking component 105' is covered with the lancet cap 106'. The lancet cap 106' and the lancet body 104' are integrally connected together by a weakened part 108'. As shown in Figs. 18 and 21, the protective cover 102' is provided to enclose a part of the lancet body 104'. Such lancet 100' is loaded into the injector 200' , and thereafter the lancet cap 106' is removed. By the removal of the lancet cap, the tip of the pricking component 105' is exposed, and thereby the lancet becomes ready for pricking.

The injector 200' shown in Fig. 19 can be used in combination with the lancet 100' to launch the lancet body with the tip of the pricking component 105' exposed. The injector 200' comprises a plunger 204'. The plunger 204' is capable of engaging with a rear end portion of the lancet body to launch the lancet body in the pricking direction (see Fig. 22). As shown in Fig. 22, the lancet 100' is loaded into the injector 200' by inserting the lancet 100' into the injector 200' through a front end opening 214' of the injector 200' . As shown in Fig. 23, when the lancet is inserted to some degree, a rear portion 116' of the lancet 100' is held by tips 264' and 266' of the plunger 204'. Subsequently, when the insertion of the lancet is continued, the plunger 204' is thrust backward so that the launching energy is stored. That is, the retraction of the plunger 204' can compress a spring (not shown) provided in the plunger 204' . This means that, when the compression of the spring is released, the plunger instantly moves forward to launch the lancet. Fig. 24 shows the injector 200' in the state where the plunger has retracted and the launching energy has been stored therein.

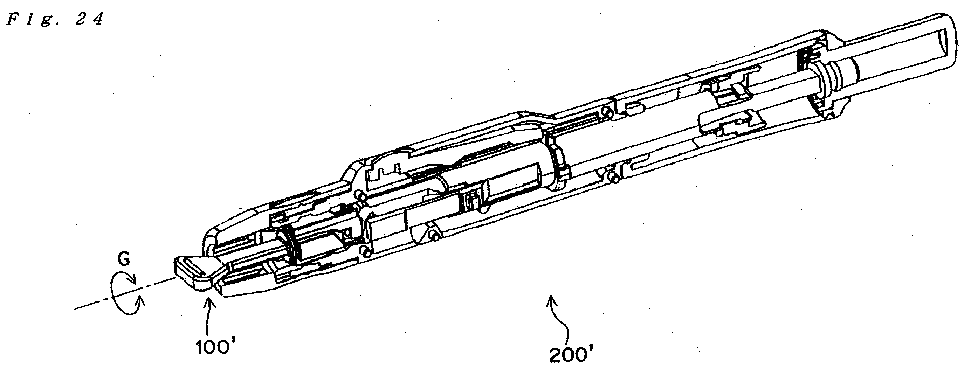

After the loading of the lancet assembly 100' into the injector 200' is completed, the lancet cap 106' is removed to expose the tip of the pricking component 105'. The removal of the lancet cap 106' will be described as follows:
As shown in Figs. 20 and 21, the lancet body 104' and the lancet cap 106' are integrally connected together by the weakened part 108' disposed between the lancet body and the lancet cap. The weakened part 108' is broken by rotating the lancet body 104' and the lancet cap 106' around the pricking component in the reverse direction to each other (see Fig. 24 in which the rotation of the lancet cap in the direction "G" is shown), whereby the removal of the lancet cap 106' can be performed. Namely, the tip of the pricking component 105' is exposed by so-called "twist off".

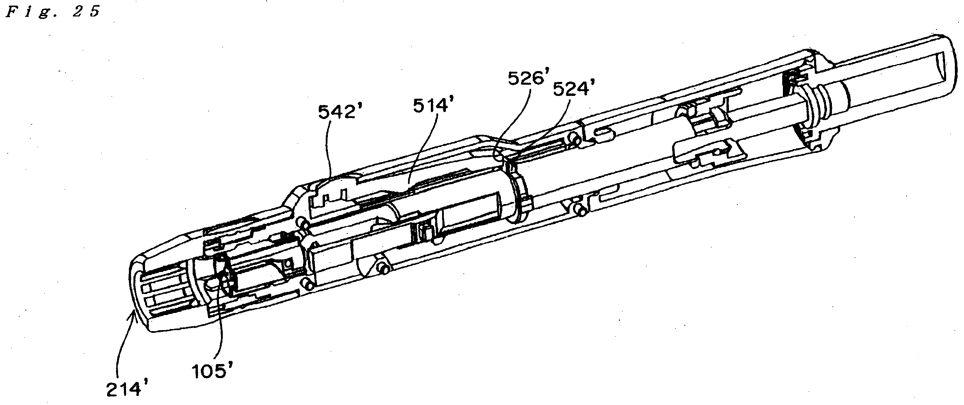

When the pricking operation is carried out, the front end opening 214' of the injector 200' is applied to a predetermined region to be pricked (for example, a finger tip). Subsequently, the press part 542' of a trigger component 514' is pushed. See Fig. 25. The pushing of the press part 542' results in an instantaneous expansion of the compressed spring, and thereby forcing the plunger 204' to move forward to prick the predetermined region by the pricking component.

With respect to the above-described pricking device, the inventors of the present application have found that the plunger of the injector, which is a major part serving to launch the lancet, still has room for the following improvements.
- The forward movement of the plunger in the pricking direction for the pricking can cause the plunger to jiggle, which can increase more pain which the subject of the blood sampling feels at the time of the pricking.
- The pricking depth at the time of pricking can be defined by the limitation on the forward movement of the plunger. The limitation, i.e., the blocking of the further forward movement of the plunger can also cause the jiggling of the plunger. Namely, when the forward movement of the plunger having been launched in the pricking direction is halfway halted, the jiggling of the tip portion of the plunger occurs due to the halting shock, which results in the more pain which the subject of the blood sampling feels at the time of the pricking.
- The plunger has an elongated form due to the required function thereof. The elongated form of the plunger, however, results in an increased volume of the injector as a whole, which will lead to unsatisfactory features of the injector in terms of a transport efficiency, a storage space and an operability thereof.

### DISCLOSURE OF THE INVENTION

### PROBLEMS TO BE SOLVED BY THE INVENTION

The present invention has been devised to address the matters to be improved described above. As such, an object of the present invention is to provide an injector equipped with a more suitable plunger.

### MEANS FOR SOLVING THE PROBLEMS

In order to achieve the above object, the present invention provides an injector for launching the lancet to provide a pricking having the features of claim 1.

One of the characterizing features of the injector according to the present invention is that the lancet-attachment portion of the plunger part has a dual cylindrical structure composed of the outer cylinder and the inner cylinder. In particular, the lancet-attachment portion has such a form that the body of the outer cylinder and the body of the inner cylinder are spaced away from each other.

The term *"cylinder"* regarding the outer and inner cylinders as used herein substantially means a part having an opening at one end thereof and a bottom portion/bottom face at the other end thereof.

In a preferred embodiment of the injector according to the present invention, the lancet-attachment portion of the plunger part is located inside of a front end opening portion of the injector casing, in which case "external body surface of the outer cylinder of the lancet-attachment portion" and "internal wall surface of the front end opening portion of the injector casing" are in close contact with or adjacent to each other. In other words, the plunger part and the injector casing are positioned with respect to each other such that substantially no clearance is provided between "outer cylinder of the lancet-attachment portion of the plunger part" and "front end opening portion of the injector casing".

When the lancet is attached to the lancet-attachment portion of the plunger part, the inner cylinder of the lancet-attachment portion separately serves with respect to the outer cylinder of the lancet-attachment portion. More specifically, during the attaching of the lancet to the lancet-attachment portion of the plunger part, the inner cylinder of the lancet-attachment portion is displaced such that it expands outward, whereas the form of the outer cylinder of the lancet-attachment portion is maintained such that the outer cylinder is not displaced.

In a preferred embodiment, the injector further comprises a spring part "A" for launching the plunger part in the pricking direction and a spring part "B" for returning the launched plunger part (i.e., the plunger part having been launched in the pricking direction), in which case the spring parts "A" and "B" are positioned in parallel with each other in a direction perpendicular to the pricking direction. In other words, the spring parts "A" and "B" are not serially positioned in a longitudinal direction of the plunger part. This means that the spring parts "A" and "B" are parallel positioned adjacent to each other such that they are in a side-by-side arrangement with each other in a direction perpendicular to the longitudinal direction of the plunger part. It is preferred in this embodiment that a rear end side portion of the plunger part is provided with two hollow portions "A" and "B", the hollow portions "A" and "B" being adjacent in parallel to each other. In this case, it is further preferred that the spring part "A" is positioned in the hollow portion "A" of the plunger part, whereas the spring part "B" is positioned in the hollow portion "B" of the plunger part. The spring part "B" additionally serves during an ejecting of the re-charged lancet at a point in time after the pricking.

In another preferred embodiment, the injector further comprises a pricking-depth adjusting mechanism, the mechanism comprising:
a disk part equipped with an anisotropically-shaped portion having side faces and a rotation axis, spaced distances "L" between the respective side faces and the rotation axis being different from each other in the anisotropically-shaped portion; and
an axis part extending through the rotation axis of the anisotropically-shaped portion,
wherein the disk part is positioned such that at least one of the side faces of the anisotropically-shaped portion takes a form of a perpendicular face with respect to the pricking direction,
wherein the axis part extends in a traverse direction of the injector,
wherein, when the plunger part moves forward for the pricking, a part of the plunger part hits the side face of the anisotropically-shaped so that a further forward movement of the plunger part is prevented,
wherein the side face which the part of the plunger part hits can be switched to the other side faces with the different spaced distances "L" by such a rotation of the disk part that the anisotropically-shaped portion is rotated about its rotation axis, and thereby the distance over which the plunger part can move forward for the pricking is changed.

It is preferred that the axis part is flexible. It is particularly preferred that the axis part exhibits flexibility in the pricking direction and the reverse direction thereof. The axis part and the disk part may be provided as an integral part, the axis part and the disk part being integrated with each other. This means that the axis part and the disk part may be provided as a preliminarily integrated single part in the injector. Furthermore, the plunger part may have a hollow portion "C" at a lateral body face thereof, and the axis part may extend through such hollow portion "C". In this case, during the forward movement of the plunger part for the pricking, a part of the lateral body face of the plunger part, which defines the hollow portion "C", can hit the side face of the anisotropically-shaped portion.

### EFFECT OF THE INVENTION

In accordance with the injector of the present invention, the lancet-attachment portion of the plunger part has the dual cylindrical structure in which the outer and inner cylinders are spaced away from each other. This enables the outer and inner cylinders to respectively have their own functions wherein the function of the outer cylinder is different from that of the inner cylinder. Specifically, upon the attaching of the lancet into the lancet-attachment portion of the plunger part, the inner cylinder provided at the tip of the plunger part makes it possible to flexibly change its geometry to facilitate a satisfactory attaching of the lancet. While on the other hand, the outer cylinder, which is positioned such that no clearance is provided with respect to the front end opening portion of the injector casing, can reduce the deviation of the pricking pathway of the lancet needle at the time of pricking. In other words, the insertion of the lancet into the inner cylinder of the lancet-attachment portion enables the inner cylinder to be displaced such that the inner cylinder expands outward, which can suitably retain the lancet in the plunger part according to the form of the inserted lancet. While on the other hand, the outer cylinder of the lancet-attachment portion, which does not undergo the displacement during the insertion of the lancet, can improve the straight movement of the lancet-attachment portion of the plunger part (i.e., straight movement of the lancet) at the time of pricking since substantially no clearance is provided between the outer cylinder and the front end opening portion of the injector casing. In this regard, if unnecessarily large clearance is provided between the lancet-attachment portion and the front end opening portion of the injector casing, the tip of the plunger part having been launched forward in no time by the force of the spring is caused to jiggle or undulate, which impairs the straight movement of the lancet. According to the present invention, substantially no clearance between "outer cylinder of the lancet-attachment portion" and "front end opening portion of the injector casing" makes it possible to prevent the jiggling or undulating of the tip of the plunger part, and thereby achieving more straight pricking path of the lancet. As a result of the more straight pricking path of the lancet, the pain felt by the person to be pricked (i.e., the subject of the blood sampling) can be effectively reduced at the time of the pricking. While not wishing to be bound by any theory, the reduced pain is believed to be attributable to a reduction of "adverse phenomenon of hollowing or scratching the pricked portion by the moving needle".

In accordance with the injector of the present invention, the two springs are positioned side-by-side in parallel with each other, and thereby achieving the shorten length of the plunger part. This enables the injector to have a compact size as a whole. As such, the injector of the present invention is excellent not only from the viewpoint of a transport efficiency and a storage space thereof because of the reduced size of the injector (especially the reduced longitudinal length of the injector), but also from the viewpoint of an operability in use thereof because of suitable size of the injector with respect to the hand or finger of the person to be pricked.

In accordance with the injector having the pricking-depth adjusting mechanism, the rotating of the disk part enables an adjusting of "pricking depth", which leads to a satisfactory operability of the injector. More specifically, while the injector is held by one hand of the user, the operation for the rotating of the disk part can be performed by a thumb of such one hand. Furthermore, due to the flexibility of the axis part, the jiggling of the pricking needle of the lancet can be reduced, the jiggling being attributed to the shock caused by the collision between a part of the plunger part and the side face of the anisotropically-shaped portion, the collision being associated with the limitation on the forward movement of the plunger part. In other words, in light of the general fact that the jiggling of the pricking needle of the lancet, i.e., the deviation of the path way of the pricking needle may occur at a point in time when the plunger part hits the anisotropically-shaped portion of the pricking-depth adjusting mechanism, an energy of such jiggling can be effectively absorbed by the axis part since the axis part to which the anisotropically-shaped portion is directly or indirectly connected has the flexibility. As such, the jiggling of the pricking needle of the lancet can be reduced at the time of pricking, which can also reduce the pain felt by the person to be pricked.

### BRIEF DESCRIPTION OF THE DRAWINGS

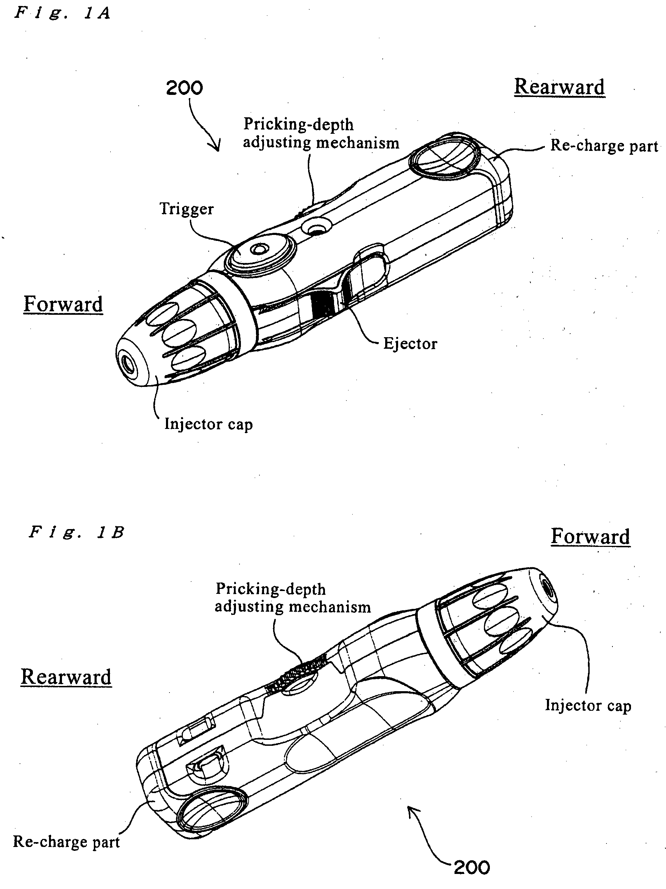

Figs. 1A and 1B are perspective views illustrating an appearance of an injector according to an embodiment of the present invention wherein Fig. 1A shows the trigger-sided view thereof, and Fig. 1B shows the back-sided view thereof with respect to the view of Fig. 1A.

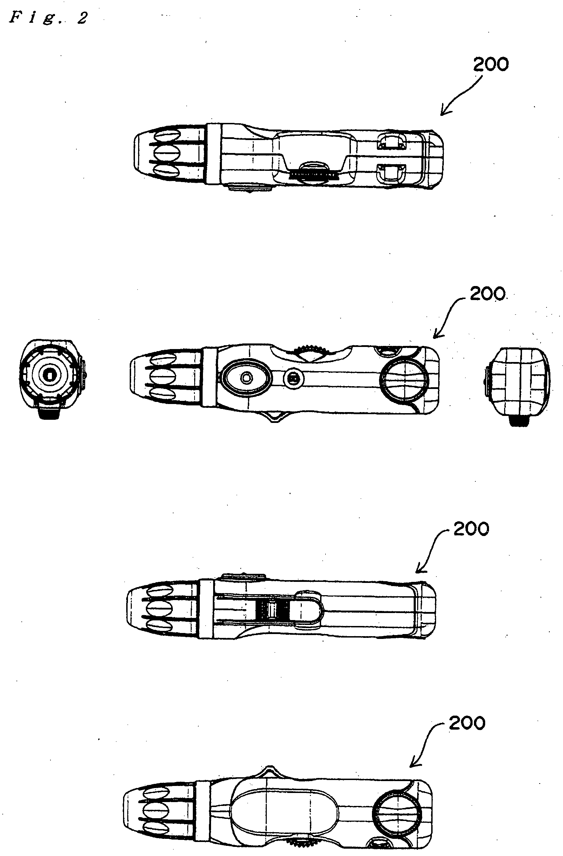

Fig 2. includes lateral-sided views and end-sided views regarding the appearance of the injector according to an embodiment of the present invention.

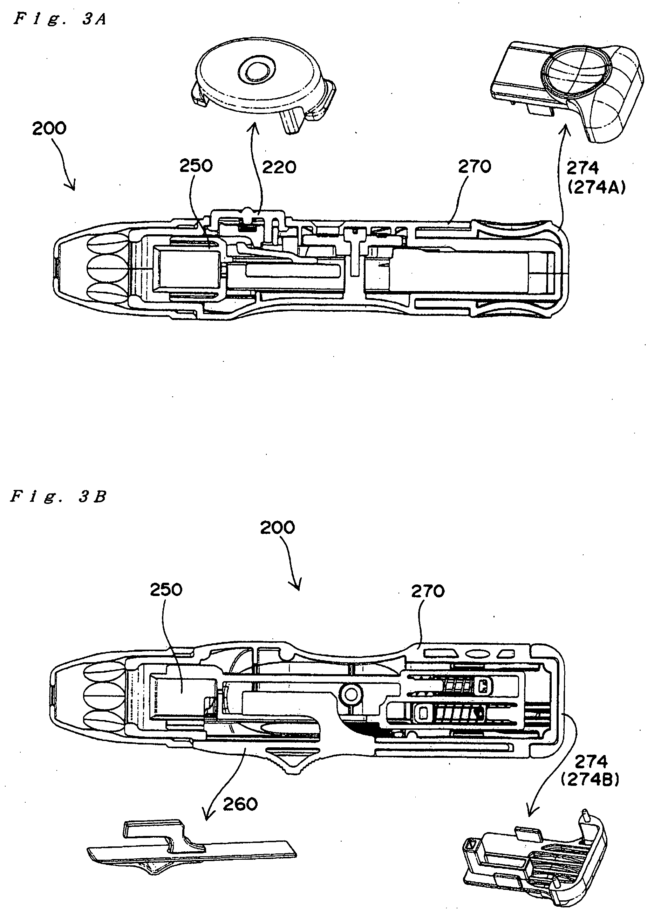

Figs. 3A and 3B are halved-sectional views illustrating the internal structures of the injector according to an embodiment of the present invention wherein Fig. 3A shows a sectional view taken by a plane for halving the trigger, and Fig. 3B shows a sectional view taken by a plane for halving the ejector.

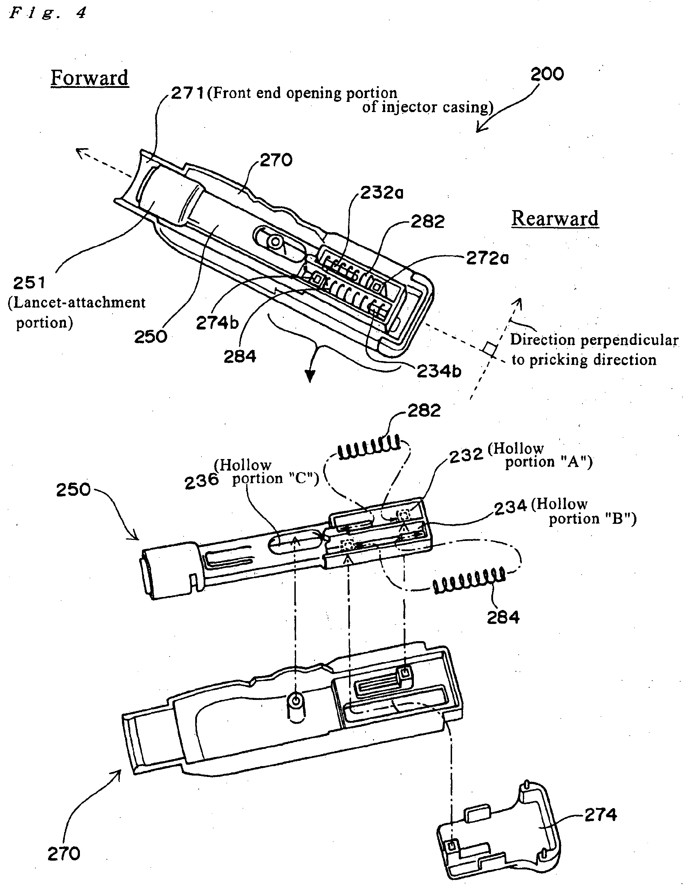

Fig 4. includes schematic and exploded views illustrating the internal structures of the injector according to an embodiment of the present invention, the injector being equipped at least with "plunger part" and "injector casing".

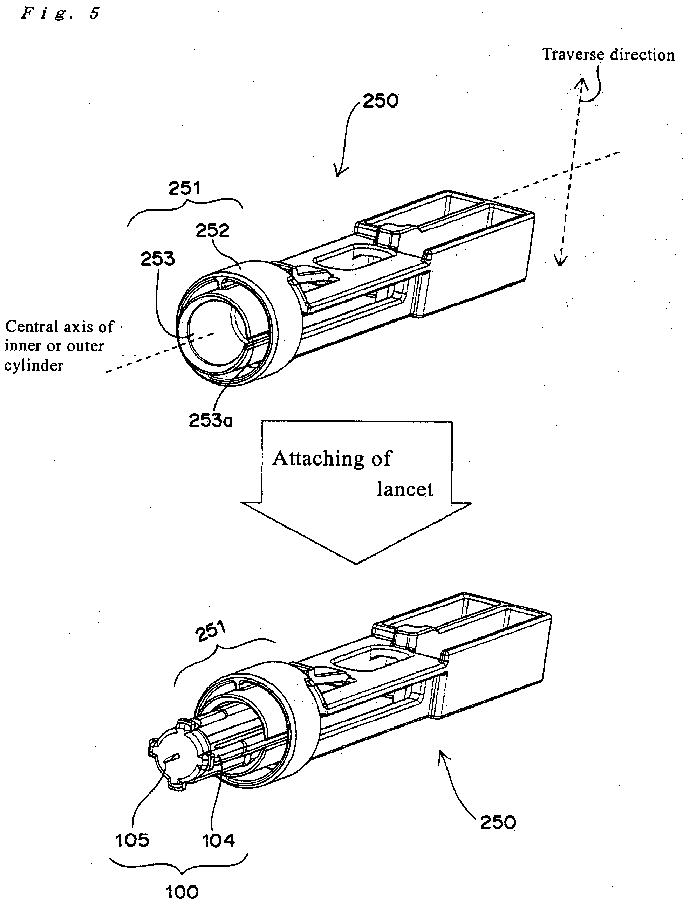

Fig 5. includes perspective views illustrating a plunger part used in the injector according to an embodiment of the present invention.

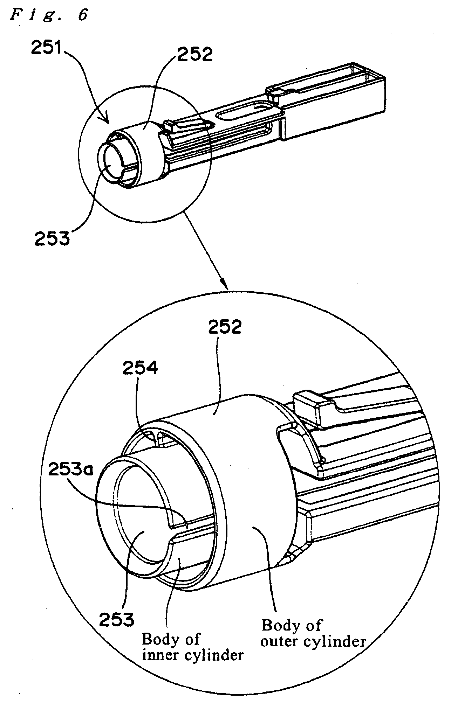

Fig 6. includes perspective and enlarged views illustrating the plunger part and an lancet-attachment portion thereof, both of which are used in the injector according to an embodiment of the present invention.

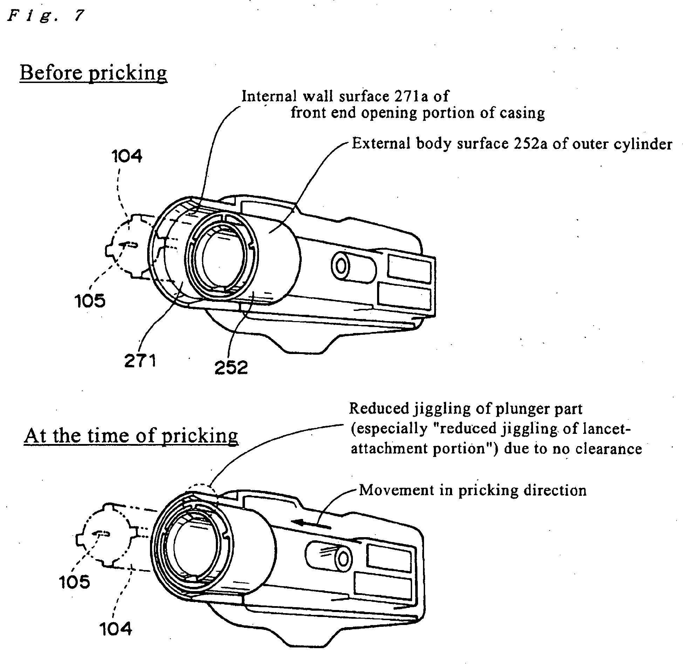

Fig 7. includes schematic views illustrating an embodiment wherein a straight pricking path of the lancet-attachment portion is achieved due to substantially no clearance between an outer cylinder of the lancet-attachment portion and a front end opening portion of an injector casing.

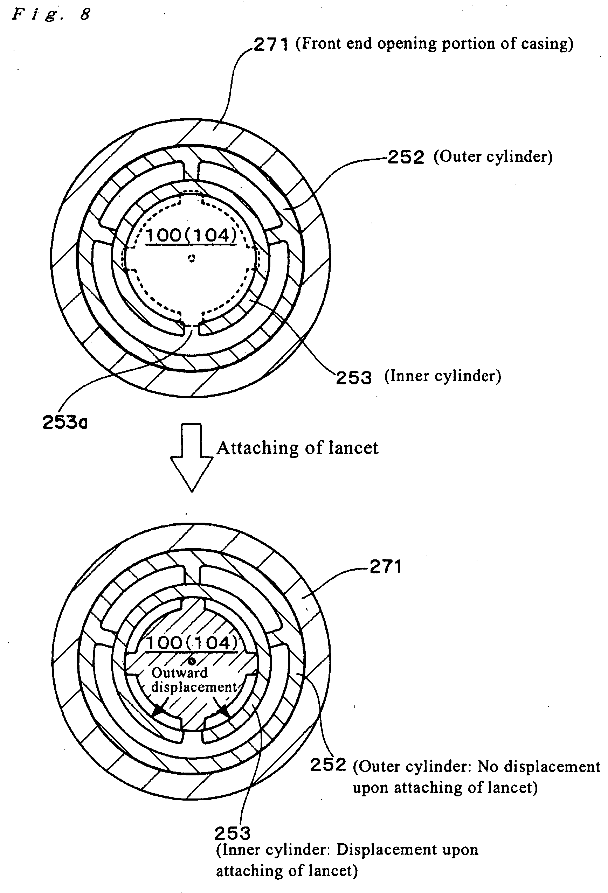

Fig 8. includes schematic views illustrating an embodiment wherein, during the attaching of the lancet into the lancet-attachment portion, an inner cylinder of the lancet-attachment portion is displaced such that it expands outward, whereas the outer cylinder of the lancet-attachment portion does not undergo the displacement such that substantially no clearance is maintained between the outer cylinder and the front end opening portion of the injector casing.

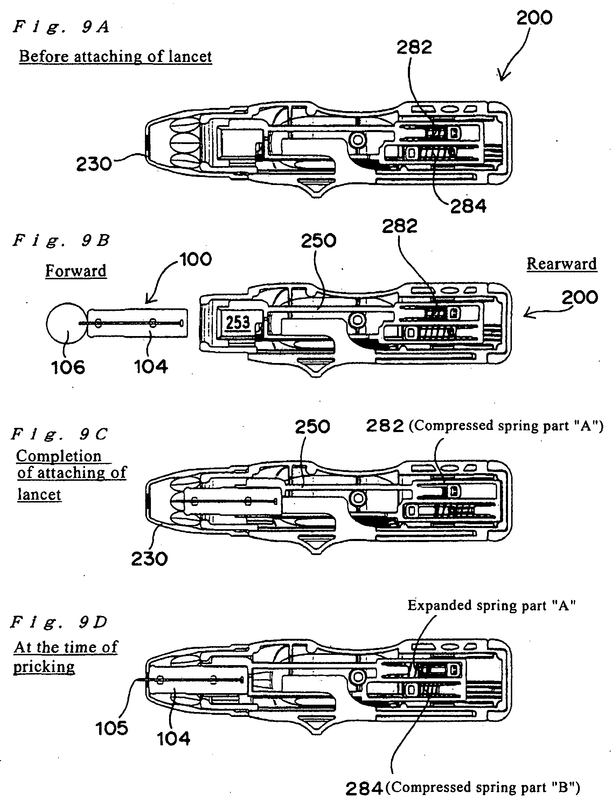

Figs. 9A to 9D are schematic cross-sectional views (especially, sectional views taken by a plane for halving the ejector) illustrating the change in the injector over time during the using thereof.

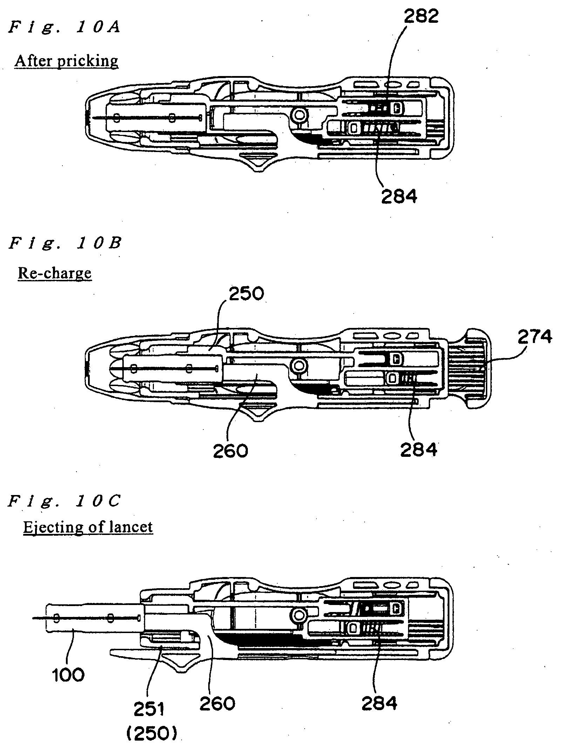

Figs. 10A to 10C are schematic cross-sectional views (especially, sectional views taken by a plane for halving the ejector) illustrating the change in the injector over time during the using thereof.

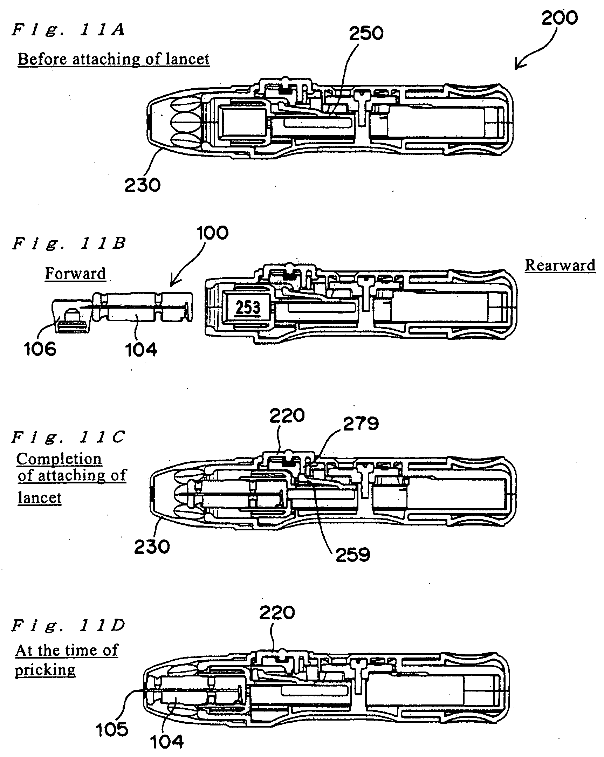

Figs. 11A to 11D are schematic cross-sectional views (especially, sectional views taken by a plane for halving the trigger) illustrating the change in the injector over time during the using thereof.

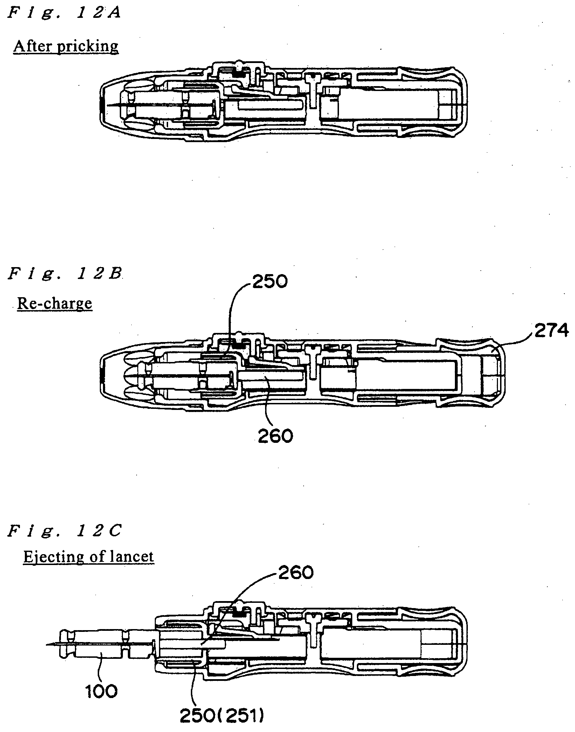

Figs. 12A to 12C are schematic cross-sectional views (especially, sectional views taken by a plane for halving the trigger) illustrating the change in the injector over time during the using thereof.

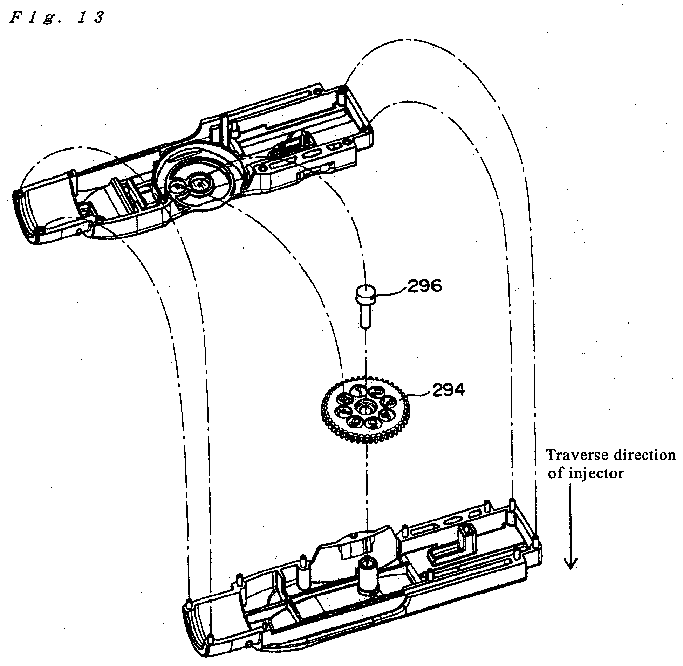

Fig. 13 is an exploded and perspective view illustrating the structure of a pricking-depth adjusting mechanism.

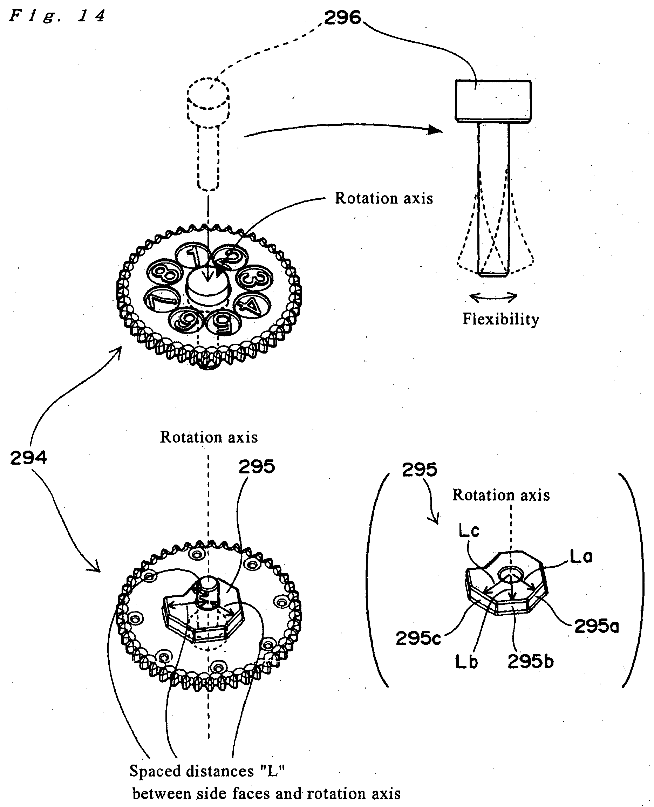

Fig. 14 includes perspective and plan views illustrating "disk part provided with an anisotropically-shaped portion" and "flexible axis part" used in the pricking-depth adjusting mechanism.

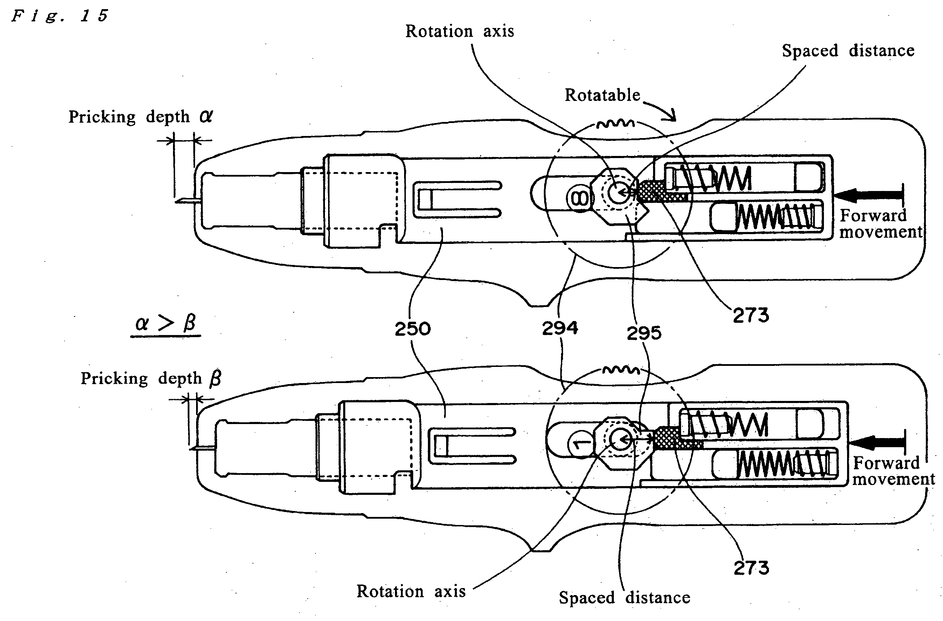

Fig. 15 includes schematic views illustrating a pricking depth being adjusted by the pricking-depth adjusting mechanism.

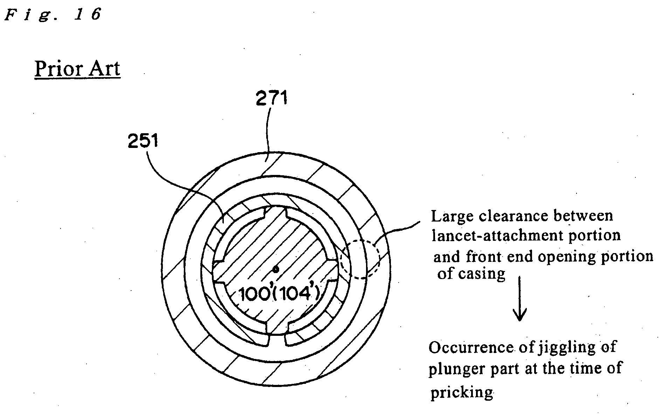

Fig. 16 is a schematic view illustrating a relative positional relationship between "lancet-attachment portion of plunger part" and "front end opening portion of injector casing" according to the prior art.

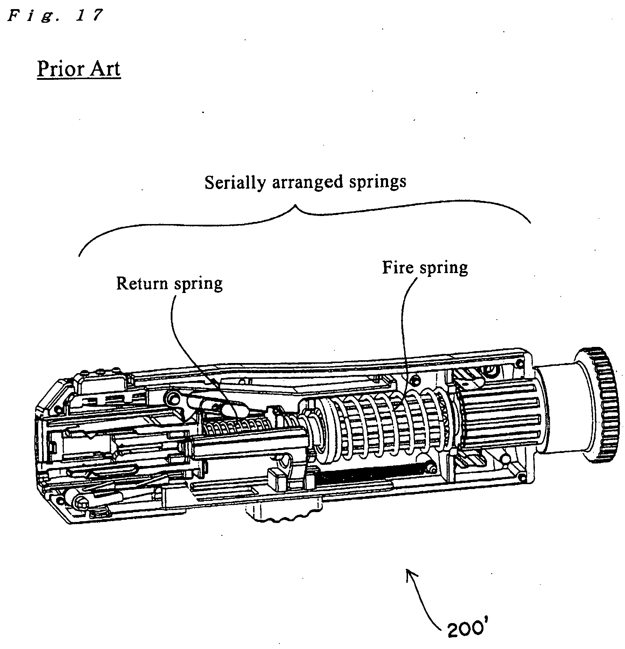

Fig. 17 is a schematic view illustrating a serial arrangement of "fire spring" and "return spring" according to the prior art.
Fig. 18 is a perspective view showing an appearance of a lancet assembly (Prior Art).
Fig. 19 is a perspective view showing an appearance of an injector (Prior Art).
Fig. 20 is a perspective view showing an appearance of a lancet (Prior Art).
Fig. 21 is a perspective view showing the lancet of Fig. 20, cut away in half so as to make it easy to understand the inside of the lancet (Prior Art).

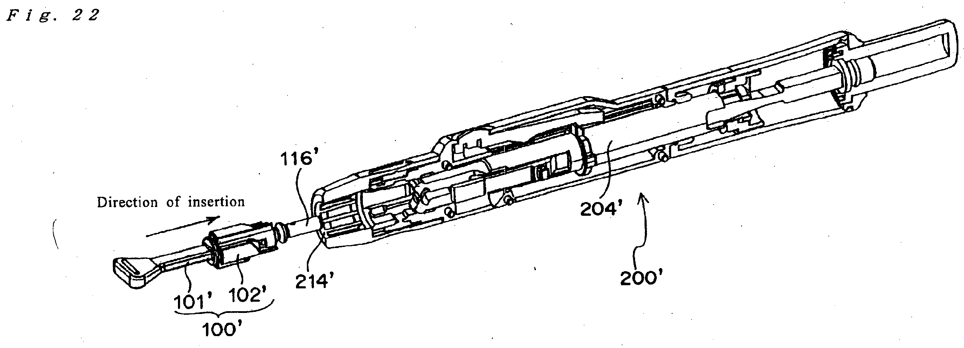

Fig. 22 is a perspective view showing the state before the lancet assembly is loaded into the injector (Prior Art).

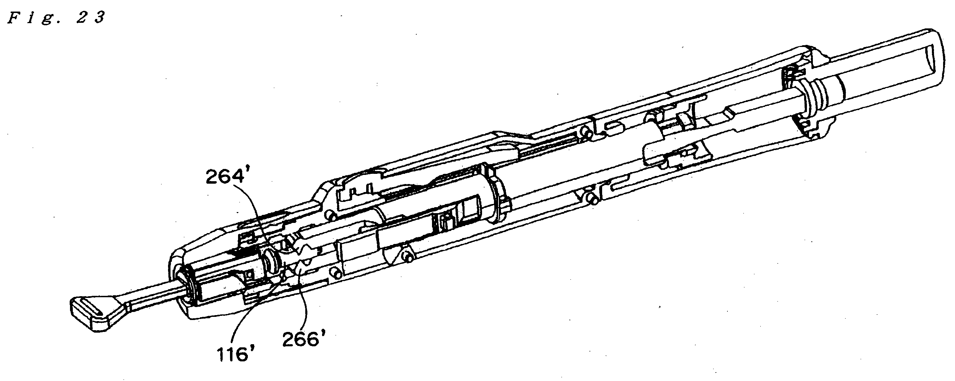

Fig. 23 is a perspective view showing the state in which the lancet is held by the tip of a plunger upon loading the lancet assembly (Prior Art).
Fig. 24 is a perspective view showing the state of completion of loading the lancet assembly wherein the plunger cannot be retracted any longer.
Fig. 25 is a perspective view showing the state in which a lancet cap has been removed and thus the lancet is ready for pricking.

### MODES FOR CARRYING OUT THE INVENTION

With reference to the accompanying drawings, an injector of the present invention will be described.

The directions as used throughout the claims and description are defined as follows: The direction from the bottom of the inner cylinder to the opening end of the inner cylinder along the central axis of the inner cylinder or in the longitudinal direction of the injector is regarded as a "forward" direction. Alternatively, the direction from the bottom of the outer cylinder to the opening end of the outer cylinder along the central axis of the outer cylinder or in the longitudinal direction of the injector is regarded as a "forward" direction. The reverse direction thereto is regarded as a "rearward"/"backward" direction. Such directions are illustrated in the drawings. The direction perpendicular to the central axis of the inner or outer cylinder or perpendicular to the longitudinal direction of the injector is regarded as a "transverse direction". It should be noted that the "forward direction" substantially corresponds to a "pricking direction" of the launched lancet in which the pricking needle or the plunger part moves for the pricking.

### <Basic Structure of Injector>

Figs. 1 to 4 illustrate an injector 200 according to an embodiment of the present invention. Figs. 1A, 1B and 2 respectively illustrate views of the appearance of the injector 200. Figs. 3 and 4 respectively illustrate the internal structure of the injector 200. As shown in Figs. 3 and 4 (especially Fig. 4), the injector 200 of the present invention at least comprises "plunger part 250" and "injector casing 270".

The plunger part 250 serves to launch the lancet in the pricking direction to provide a pricking. The plunger part 250 is located in the interior of the casing 270. Especially as shown in Fig. 4, a lancet-attachment portion 251 of the plunger part is provided at the front side of the injector. As such, the plunger part 250 is located within the injector casing 270 such that the lancet-attachment portion 251 of the plunger part is positioned inside of a front end opening portion 271 of the casing 270.

The plunger part 250 may be made of any suitable kinds of resin materials as long as they are used for a plunger of a pricking device in general. Similarly, the injector casing 270 may be made of any suitable kinds of resin materials as long as they are used for a casing/housing of a pricking device in general.

As shown in Fig. 4, the tip of the plunger part 250 is provided with the lancet-attachment portion 251. During the use of the injector, the lancet 100 is loaded into the lancet-attachment portion 251 as shown in Fig. 5. More specifically, the attaching of the lancet 100 with respect to the plunger part 250 is performed by an insertion of a lancet body 104 of the lancet 100 into the lancet-attachment portion 251 of the plunger part. Such insertion of the lancet causes the plunger part 250 to be retracted within the casing 270 since a pressing force due to the insertion of the lancet 100 is applied on the plunger part 250. As a result, a force necessary for the pricking is stored in the retracted plunger part 250. More specifically, a fire spring provided in the plunger part is compressed as the insertion of the lancet proceeds, and thereby the force necessary for launching the lancet is stored in the plunger part. At a point in time when the lancet can be ready for the launching after the completion of the attaching of the lancet to the plunger part, a lancet cap is removed from the lancet to expose a pricking needle. Thereafter, when the compressed state of the spring is released, the plunger part (i.e., the lancet with its pricking needle exposed, the lancet being attached to the plunger part) is launched in the pricking direction to provide the pricking.

### (Dual Cylindrical Structure of Plunger Part)

The plunger part 250 of the injector according to an embodiment of the present invention has a dual cylindrical structure in which the lancet-attachment portion 251 is provided at the tip of the plunger part, the lancet-attachment portion 251 being composed of an outer cylinder 252 and an inner cylinder 253. Specifically, the respective bodies of the outer cylinder 252 and the inner cylinder 253 are spaced away from each other in the lancet-attachment portion 251 at the tip of the plunger part, as shown in Fig. 6. More specifically, the body of the outer cylinder and the body of the inner cylinder are spaced away from each other in a traverse direction of the injector. By way of example, the spaced distance between the bodies of the outer and inner cylinders may be in the approximate range of 1.5 mm to 4 mm, especially in the approximate range of 1.5 mm to 2 mm. Such spacing between the bodies of the outer and inner cylinders 252,253 enables the outer and inner cylinders 252,253 to respectively possess their own functions. For example, the outer cylinder 252 of the lancet-attachment portion 251 can contribute to an improvement of the straight pricking movement of the plunger part, whereas the inner cylinder 253 of the lancet-attachment portion 251 can contribute to a favorable loading/insertion of the lancet. In this way, the outer and inner cylinders respectively have their own functions in the lancet-attachment portion of the injector according to an embodiment of the present invention.

Now, "outer cylinder" will be firstly described in more detail. The outer cylinder 252 of the lancet-attachment portion is provided such that substantially no clearance is provided between the outer cylinder and the front end opening portion 271 of the injector casing, as shown in Figs. 4 and 7. In particular, "external body surface 252a of the outer cylinder 252 of the lancet-attachment portion" and "internal wall surface 271a of the front end opening portion 271 of the injector casing" are in close contact with or adjacent to each other, as shown in Figs. 4 and 7. The phrase *"close contact with* or *adjacent* to *each other"* as used herein substantially means that a distance between the external body surface 252a and the internal wall surface 271a is in the approximate range of 0 to 1.4 mm, preferably in the approximate range of 0 to 1.0 mm, more preferably in the approximate range of 0 to 0.5 mm (for example, in the approximate range of 0 to 0.2 mm). As such, substantially no clearance between "outer cylinder 252 of the lancet-attachment portion" and "front end opening portion 271 of the injector casing" enables the pathway of the plunger part to become more straight for the pricking. In this regard, if unnecessarily large clearance is provided between the lancet-attachment portion and the front end opening portion of the injector casing (e.g., in a case of an embodiment of Fig. 16 illustrating the lancet-attachment portion of the prior art), the tip of the plunger part having been launched forward in no time by the force of the spring is caused to jiggle or undulate, which impairs the straight movement of the lancet. According to the present invention, substantially no clearance between "outer cylinder 252 of the lancet-attachment portion" and "front end opening portion 271 of the injector casing" makes it possible to prevent the jiggling or undulating of the tip of the plunger part, and thereby achieving the more straight moving path of the plunger part (especially, the more straight moving path of the lancet-attachment portion 251 of the plunger part). See Fig. 7. The more straight pricking path can effectively reduce the pain felt by the person to be pricked (i.e., the subject of the blood sampling) at the time of the pricking. While not wishing to be bound by any theory, the reduced pain is believed to be attributable to a reduction of "adverse phenomenon of hollowing or scratching the pricked portion by the moving needle".

The other cylinder of the lancet-attachment portion, i.e., "inner cylinder 253" is positioned inside of the outer cylinder such that the inner and outer cylinders are spaced away from each other. The body of the inner cylinder has a cutout portion (see the cutout portion illustrated by reference numeral "253a" in Figs. 5 and 6). As such, the inner cylinder 253 is deformable during the insertion of the lancet into the inner cylinder 253. In this regard, the inner cylinder is capable of expanding outward when the lancet is inserted into the inner cylinder 253. The presence of the cutout portion 253a facilitates the outward expansion of the inner cylinder in the traverse direction of the injector. A space for allowing the outward expansion of the inner cylinder is provided outside of the inner cylinder, and thereby the large degree of freedom on the deformation of the inner cylinder is achieved. As a result, a favorable loading of the lancet into the lancet-attachment portion can be achieved, the favorable loading being more appropriate for the lancet body of the lancet (see Fig. 8).

As such, the inner cylinder can be displaced to expand outward upon the loading of the lancet into the inner cylinder, and thereby making it possible to suitably secure the lancet to the plunger part. While on the other hand, the outer cylinder, which cannot be displaced upon the loading of the lancet, and thus keeps substantially no clearance with respect to the front end opening portion of the injector casing, can eventually contribute to the suitable improvement in the straight pricking movement of the lancet-attachment portion (i.e., lancet). Fig. 8 schematically shows such an embodiment that the inner cylinder 253 can be displaced to expand outward upon the loading of the lancet, and the outer cylinder cannot be displaced upon the loading of the lancet so that it keeps substantially no clearance with respect to the front end opening portion of the injector casing.

### (Spring of Plunger Part)

The plunger part 250 of the injector according to an embodiment of the present invention has a unique arrangement of springs, which is unlike the prior art. Specifically, as shown in Fig. 4, a spring part "A" (282) for launching the plunger part in the pricking direction and a spring part **"**B" (284) for returning the launched plunger part are provided in the plunger part. The spring part "A" (282) and the spring part "B" (284) are positioned in parallel with each other in a direction perpendicular to the pricking direction. This means that the spring parts "A" and "B" (282, 284) are positioned side-by-side with each other in the short dimension direction of the injector. According to the prior art (see Fig. 17, for example), "fire spring for launching the plunger part in the pricking direction" and "return spring for returning the launched plunger" are provided in a serial arrangement with each other in the plunger. Especially, according to the prior art, the fire spring and the return spring are located adjacent to each other in the pricking direction. This means that the injector according to the prior art necessarily has a long length which at least corresponds to the dimension of "serial arrangement of the fire and return springs". In contrast, the injector according to the present invention has a shorter length of the plunger part since the two springs of the plunger part are positioned side-by-side with each other in parallel. This results in a compact size of the injector as a whole. By way of example, the longitudinal length (i.e., long dimension length) of the injector can be shorter than that of the prior art by about 10% to about 40%, preferably about 20% to 40% (for example, 30% to 40%). The shorter length of the injector in the longitudinal direction thereof leads to a satisfactory transport efficiency and a satisfactory storage space, and also leads to a satisfactory operability in use thereof because a suitable size of the injector can be provided with respect to the hand or finger of the person to be pricked.

Each of the spring parts "A" and "B" (282, 284) is provided such that it is housed in a hollow portion of the plunger part. This means that the injector of the present invention has been created by an inventive approach to a form of the plunger part so as to suitably realize the parallel arrangement of the spring parts. Specifically, as shown in Fig. 4, the plunger part 250 has two hollow portions "A" (232) and "B" (234) at a rear end side portion thereof, the hollow portions "A" and "B" being adjacent in parallel to each other in the traverse direction of the plunger part (i.e., in a direction perpendicular to the pricking direction), in which case the spring part "A" (282) is positioned in the hollow portion "A" (232) of the plunger part, whereas the spring part "B" (284) is positioned in the hollow portion "B" (234) of the plunger part. The parallel arrangement of the two spring parts makes it possible to promote effective utilization of the internal space of the injector casing 270, which results in a compact size of the injector as a whole.

More concrete arrangement of the spring parts in the hollow portions will be described. As shown in Fig. 4 (especially, the upper illustration thereof), the spring "A" (282) is provided such that the front end thereof is attached to "front spring-attachment portion 232a of the hollow portion "A" (i.e., plunger-sided portion)", whereas the rear end thereof can be in contact with "casing boss 272a protruding and extending in the traverse direction of the hollow part "A" (i.e., casing-sided portion)". As shown in Fig. 4 (especially, the upper illustration thereof), the spring "B" (284) is provided such that the front end thereof can be in contact with "boss 274b of a casing re-charge part 274, the boss protruding and extending in the traverse direction of the hollow part "B" (i.e., casing-sided portion)", whereas the rear end thereof is attached to "rear spring-attachment portion 234b of the hollow portion "B" (i.e., plunger-sided portion)".

In the following, the embodiments of use of the injector 200 will be described with reference to Figs. 9 to 12 to give a better understanding of the respective functions of the spring parts "A" and "B". "Figs. 9A to 9D / Figs. 10A to 10C" or "Figs. 11A to 11D / Figs. 12A to 12C" show the changes of the injector 200 over time in numerical order. It is noted that the figures indicated by the same alphabet among "Figs. 9A to 9D / Figs. 10A to 10C" or "Figs. 11A to 11D / Figs. 12A to 12C" respectively show the same state.

First, as shown in Figs. 9A and 9B, at a point in time before the lancet 100 is loaded (i.e., at a point in time before pricking), the spring part "A" (282) provided in the plunger part is not compressed. For use of the injector, an injector cap 230 is removed from the injector, and thereafter the lancet 100 is inserted into the inner cylinder 253 of the lancet-attachment portion provided at the tip of the plunger part. During the insertion of the lancet 100, the plunger part 250 is forced to move backward. Specifically, the insertion of the lancet 100 acts to press the plunger part 250, which results in a retracting of the plunger part 250. As shown in Figs. 9B and 9C, the retracting of the plunger part 250 causes the spring part "A"

(282) to be compressed and the necessary force for the launching is stored in the plunger part. Eventually, the compressed state of the spring part "A" (282) is kept by an engagement between an engaging portion of the plunger part and an engaged portion of the trigger. For example, the engagement between the engaging portion 259 of the plunger part and the engaged portion 279 of the trigger is performed as shown in Fig. 11C. When such keeping of the compressed state of the spring part "A" (282) by the engagement is completed, the injector becomes ready for pricking.

At a point in time after the injector becomes ready for pricking, the lancet cap 106 is removed from the lancet to expose the pricking needle. After the exposure of the pricking needle, the injector cap 230 is re-attached to the injector. For the purpose of the pricking, the trigger 220 (see Fig. 11C) is pressed toward the interior of the injector from the outside, and thereby releasing the engagement between "engaging portion of the plunger part" and "engaged portion of the trigger". This results in an instantaneous expansion of the compressed spring "A" (282), and thereby the lancet body 104 with the pricking needle 105 exposed is launched forward in the pricking direction (Fig. 9D). At the exact time of pricking, the spring part "A" (282) expands to the maximum whereas the spring part "B" (284) is compressed, as shown in Fig. 9D. After the pricking, therefore, the spring part "A" (282) attempts to returns to its original shape whereas the compressed spring part "B" (284) serves to press the plunger part 250 backward to returns to its original position. This causes the plunger part (hence the lancet body 104/the pricking needle 105) to be retracted quickly. The state after the pricking is shown in Fig. 10A and also Fig. 12A.

As will be appreciated from the above embodiment of the injector during the use thereof, the spring part "A" (282) serves to mainly launch the plunger part in the pricking direction, whereas the spring part "B" (284) serves to mainly rerun the launched plunger part. As such, the spring parts "A" and "B" (282, 284), which are located adjacent in parallel to each other, respectively have their own functions which are different from each other.

At a point in time after the pricking, the lancet 100 is ejected from the injector. Specifically, as shown in Figs. 10B and 10C, the used plunger part is forced to move backward (i.e., the plunger part is re-charged), and thereafter a forward sliding movement of an ejector 260 is performed so as to detach the lancet 100 from the plunger part 250, i.e., eject the lancet 100 from the injector. As seen from illustrations of Figs. 10B and 10C, the spring part "B" (284) additionally can function upon the re-charging and the ejecting of the lancet at a point in time after the pricking. Specifically, when a re-charge part 274 of the casing is moved backward to perform the re-charge, the spring part "B" (284) gives a moderate repulsion/resistance. And also, during the ejecting of the lancet, the spring part "B" (284) effectively serves to remove the lancet 100 from the plunger part 251. More specifically, in the light of the fact that the plunger part receives the forward-moving force due to the pressing of the lancet by the ejector, the spring part "B" (284) can prevent the plunger part 250 from greatly moving forward beyond necessity.

### (Pricking-depth Adjusting Mechanism of Plunger Part)

The plunger part 250 of the injector according to an embodiment of the present invention has a unique "Pricking-depth Adjusting Mechanism" which is unlike the prior art. The pricking-depth adjusting mechanism according to the present invention at least comprises a disk part 294 and an axis part 296, as shown in Figs. 13 and 14. Especially as shown in Fig. 14, the disk part 294 is equipped with an anisotropically-shaped portion 295 having side faces and a rotation axis, spaced distances "L" between the respective side faces and the rotation axis being different from each other in the anisotropically-shaped portion. The axis part 296 is provided such that it extends through the rotation axis of the anisotropically-shaped portion 295.

In the pricking-depth adjusting mechanism, the disk part 294 is positioned such that at least one of the side faces (295a, 295b, 295c, 295d, ··· ) of the anisotropically-shaped portion 295 takes a form of a perpendicular face with respect to the pricking direction, and also the axis part 296 extends along the traverse direction of the injector. When the plunger part 250 moves forward for the pricking, a part of the plunger part hits the side face of the anisotropically-shaped portion so that a further forward movement of the plunger part is prevented. As such, the side face which a part of the plunger part 250 (e.g., a part of the body of the plunger part) hits can be switched to the other side faces with the different spaced distances "L" by such a rotation of the disk part 294 that the anisotropically-shaped portion 295 is rotated about its rotation axis, and thereby the distance over which the plunger part can move forward for the pricking is changed. See Fig. 15. The forward movement of the plunger part can be halfway halted by the side faces (295a, 295b, 295c, 295d, ··· ) of the anisotropically-shaped portion 295. The change from one side face to the other side face enables the halted position to be changed, which results in change of the distance over which the plunger part can move forward. As a result, the exposed length of the pricking needle from the injector cap can be changed. As shown in Fig. 14, the scale markings on the disk part 294 has a correspondence relationship with respect to the side faces with the different spaced distances "L". According to the scale markings on the disk part 294, the user can switch the side face of the anisotropically-shaped portion 295 to the other side faces with the different spaced distances "L", the side face being hit by a part of the plunger part.

It is preferred that the axis part 296 is flexible. It is particularly preferred that the axis part 296 is capable of be flexed in the pricking direction and the reverse direction thereof. Due to the flexibility of the axis part, the jiggling of the pricking needle of the lancet can be effectively reduced, jiggling being attributed to the halting of the forward movement of the plunger part. Specifically, the plunger part with the attached lancet having the exposed pricking needle 105 hits the anisotropically-shaped portion at the time of pricking. When the axis part to which the anisotropically-shaped portion is connected preferably has the flexibility, an energy occurred upon the hitting of the plunger part can be effectively absorbed by the axis part. As a result, a shock energy transmitted to the pricking needle at a point in time of the hitting of the plunger part can be reduced, which leads to a reduction in the jiggling of the pricking needle of the lancet.

It is preferred that the flexible axis part 296 is made of a soft material such as a soft resin material and an elastic material. By way of example, the flexible axis part 296 may be made of at least one kind of material selected from the group consisting of silicon, polypropylene, polyethylene, rubber, polyester elastomer, for example. In the case where the injector has the pricking-depth adjusting mechanism, the axis part 296 and the disk part 294 may be integrally configured with respect to each other. Namely, the axis part 296 and the disk part 294 may be provided as a preliminarily integrated part, i.e., single integral part. In this case, the disk part and/or its anisotropically-shaped portion, together with the axis part may also be flexible. In the case of the integrated axis and disk parts 296, 294, the axis part 296 and the disk part 294 can be produced from the soft material by an integral molding process. The integrated axis and disk parts 296, 294 thus produced can be provided as a single part made of the soft material.

Preferably, the plunger part 250 has a hollow portion "C" (indicated by reference numeral "236" in Fig. 4, for example) at a lateral body face thereof. Preferably, the axis part 296 may extend through the hollow portion "C". In this regard, it is preferred that the tip of the axis part 296 extending across the hollow portion "C" is positioned in a hole portion provided in the internal surface of the casing. When the plunger part moves forward for the pricking, it is preferred that a part of the lateral body of the plunger part, which part defines the hollow portion "C" (the part being indicated by reference numeral "273" in Fig. 15), hits the anisotropically-shaped portion 295, and thereby the forward movement of the plunger part is halted. According to this embodiment, there can be provided smaller volume occupied by the pricking-depth adjusting mechanism in the injector. As seen from figures, a larger size of the injector is not particularly required due to the smaller size of the pricking-depth adjusting mechanism, which eventually leads to a compact size of the injector as a whole.

Although some embodiments of the present invention have been hereinbefore described, such embodiments are only for illustrative purpose as typical examples, and thus the present invention is not limited to these embodiments. It will be readily appreciated by those skilled in the art that various modifications are possible without departing from the scope of the invention.

In this regard, the respective bodies of the outer cylinder 252 and the inner cylinder 253 are spaced away from each other in the lancet-attachment portion 251. As long as the displacement of the inner cylinder upon the attaching of the lancet is not inhibited, there may be provided a rib (indicated by reference numeral "254" in Fig. 6, for example) which serves to locally connect between the body of the outer cylinder and the body of the inner cylinder. The presence of the rib enables the inner cylinder located inside of the outer cylinder to be held in more stable state.

### INDUSTRIAL APPLICABILITY

The injector of the present invention is capable of launching the lancet for the purpose of serving to prick. Thus, such injector can be used as a blood-sampling device in combination with the lancet.

### CROSS REFERENCE TO RELATED PATENT APPLICATION

The present application claims the right of priority of Japanese Patent Application No. 2012-114721 (filed on May 18, 2012, the title of the invention: "INJECTOR").

### REFERENCE NUMERALS

100···Lancet, 104···Lancet body, 105···Pricking needle, 106··· Lancet cap, 200···Injector, 20···Trigger, 230···Injector cap, 232···Hollow portion "A" provided in body of plunger part, 232a···Spring-attachment portion located at forward side of hollow part "B" (plunger-sided attachment portion), 234··· Hollow portion "B" provided in body of plunger part, 234b··· Spring-attachment portion located at rearward side of hollow part "B" (plunger-sided attachment portion), 236···Hollow portion "C" provided in body of plunger part, 250···Plunger part, 251···Lancet-attachment portion, 252···Outer cylinder of lancet-attachment portion, 252a···External body surface of outer cylinder, 253···Inner cylinder of lancet-attachment portion, 253a···Cutout of inner cylinder, 254···Rib part, 259···Engaging portion of plunger part, 260···Ejector, 270··· Injector casing, 271a···Internal surface of front end opening portion of injector casing, 274···Recharge part of casing, 274A···Halved member of recharge part, 274B···Halved member of recharge part, 274b···Protrusion of recharge part, 279··· Engaged portion of trigger, 282···Spring part "A", 284··· Spring part "B", 294···Disk part of pricking-depth adjusting mechanism, 295···Anisotropically-shaped portion of disk part, 296···Axis part of pricking-depth adjusting mechanism, 100' ···Lancet assembly, 101' ···Lancet, 102' ···Protective cover, 104' ···Lancet body, 105' ···Pricking part, 106' ··· Lancet cap, 108' ···weakened part, 200' ···injector, 204' ··· plunger, 214' ···Front end opening of injector, 264' , 266'··· Tip of plunger, 514' ···Trigger component, 542' ···Press part of trigger component

## Claims

1. An injector for launching the lancet to provide a pricking, the injector comprising:
a plunger part (250) capable of launching the lancet (100) in a pricking direction; and
an injector casing (270), the plunger part (250) being located in an interior space of the casing (270),
wherein the plunger part (250) comprises a lancet-attachment portion (251) in its tip,
**characterized in that**
the lancet-attachment portion (251) has a dual cylindrical structure composed of an outer cylinder (252) and an inner cylinder (253) such that respective bodies of the outer (252) and inner (253) cylinders are spaced away from each other,
wherein, when the lancet (100) is attached to the lancet-attachment portion (251), the inner cylinder (253) of the lancet-attachment portion (251) is displaced to expand outward, whereas the outer cylinder (252) of the lancet-attachment portion is not displaced.

2. The injector according to claim 1, wherein the lancet-attachment portion (251) of the plunger part (250) is located inside of a front end opening portion of the injector casing (270), and
wherein an external body surface of the outer cylinder of the lancet-attachment portion (251) and an internal wall surface of the front end opening portion of the injector casing (270) are in close contact with or adjacent to each other.

3. The injector according to any one of claims 1 to 2, further comprising:
a spring part "A" (282) for launching the plunger part (250) in the pricking direction; and
a spring part "B" (284) for returning the launched plunger part (250),
wherein the spring parts "A" and "B" are positioned in parallel with each other in a direction perpendicular to the pricking direction.

4. The injector according to claim 3, wherein a rear end side portion of the plunger part (250) is provided with two hollow portions "A" and "B", the hollow portions "A" and "B" being adjacent in parallel to each other,
wherein the spring part "A" is positioned in the hollow portion "A" of the plunger part, whereas the spring part "B" is positioned in the hollow portion "B" of the plunger part.

5. The injector according to any one of claims 1 to 4, further comprising a pricking-depth adjusting mechanism, the mechanism comprising:
a disk part (294) provided with an anisotropically-shaped portion having side faces (295a/b/c/d) and a rotation axis, spaced distances "L" between the respective side faces and the rotation axis being different from each other in the anisotropically-shaped portion; and
an axis part (296) extending through the rotation axis of the anisotropically-shaped portion,
wherein the disk part (294) is positioned such that at least one of the side faces of the anisotropically-shaped portion takes a form of a perpendicular face with respect to the pricking direction,
wherein the axis part (296) extends in a traverse direction of the injector,
wherein, when the plunger part (250) moves forward for the pricking, a part of the plunger part (250) hits the side face of the anisotropically-shaped portion so that a further forward movement of the plunger part (250) is prevented, and
wherein the side face which the part of the plunger part hits can be switched to the other side faces with the different spaced distances "L" by such a rotation of the disk part that the anisotropically-shaped, portion is rotated about the rotation axis thereof, and thereby the distance over which the plunger part can move forward for the pricking is changed.

6. The injector according to claim 5, wherein the axis part (296) is flexible.

7. The injector according to claim 6, wherein the axis part (296) exhibits flexibility in the pricking direction and a reverse direction thereof.

8. The injector according to any one of claims 5 to 7, wherein the axis part and the disk part are provided as an integral part, the axis part and the disk part being integrated with each other.

## Patentansprüche

1. Injektor zum Starten der Lanzette, um ein Einstechen vorzusehen, wobei der Injektor umfasst:
einen Kolbenteil (250), der zum Starten der Lanzette (100) in eine Einstechrichtung imstande ist; und
ein Injektor-Gehäuse (270), wobei sich der Kolbenteil (250) in einem Innenraum des Gehäuses (270) befindet,
wobei der Kolbenteil (250) einen Lanzette-Anbringungsabschnitt (251) in seiner Spitze umfasst,
**dadurch gekennzeichnet, dass**
der Lanzette-Anbringungsabschnitt (251) eine Doppelzylinderstruktur aufweist, die aus einem Außenzylinder (252) und einem Innenzylinder gebildet ist, derart dass entsprechende Körper des Außenzylinders (252) und des Innenzylinders (253) voneinander weg beabstandet sind,
wobei, wenn die Lanzette (100) an dem Lanzette-Anbringungsabschnitt (251) angebracht ist, der Innenzylinder (253) des Lanzette-Anbringungsabschnitts (251) verschoben wird, um sich nach außen auszudehnen, wohingegen der Außenzylinder (252) des Lanzette-Anbringungsabschnitts nicht verschoben wird.

2. Injektor nach Anspruch 1, wobei sich der Lanzette-Anbringungsabschnitt (251) des Kolbenteils (250) im Inneren eines Vorderes-Ende-Öffnungsabschnitts des Injektorgehäuses (270) befindet, und
wobei eine Außenkörperoberfläche des Außenzylinders des Lanzette-Anbringungsabschnitts (251) und eine Innenwandoberfläche des Vorderes-Ende-Öffnungsabschnitts des Injektorgehäuses (270) in engem Kontakt miteinander oder aneinander angrenzend sind.

3. Injektor nach einem der Ansprüche 1 bis 2, ferner mit:
einem Federteil "A" (282) zum Starten des Kolbenteils (250) in die Einstechrichtung; und
einem Federteil "B" (284) zum Zurückbringen des gestarteten Kolbenteils (250),
wobei die Federteile "A" und "B" in eine Richtung senkrecht zu der Einstechrichtung parallel zueinander positioniert sind.

4. Injektor nach Anspruch 3, wobei ein Hinteres-Ende-Seitenabschnitt des Kolbenteils (250) mit zwei hohlen Abschnitten "A" und "B" versehen ist, wobei die hohlen Abschnitte "A" und "B" parallel zueinander angrenzend sind,
wobei der Federteil "A" in dem hohlen Abschnitt "A" des Kolbenteils positioniert ist, wohingegen der Federteil "B" in dem hohlen Abschnitt "B" des Kolbenteils positioniert ist.

5. Injektor nach einem der Ansprüche 1 bis 4, ferner mit einem Einstechtiefe-Einstellmechanismus, wobei der Mechanismus umfasst:
einen Scheibenteil (294), der mit einem anisotrop-geformten Abschnitt versehen ist, der Seitenflächen (295a/b/c/d) und eine Rotationsachse aufweist, wobei sich Abstandsentfernungen "L" zwischen den entsprechenden Seitenflächen und der Rotationsachse voneinander in dem anisotrop-geformten Abschnitt unterscheiden; und
einen Achsenteil (296), der sich durch die Rotationsachse des anisotrop-geformten Abschnitts erstreckt,
wobei der Scheibenteil (294) derart positioniert ist, dass zumindest eine der Seitenflächen des anisotrop-geformten Abschnitts eine Form einer senkrechten Fläche in Bezug auf die Einstechrichtung annimmt,
wobei sich der Achsenteil (296) in eine Querrichtung des Injektors erstreckt,
wobei, wenn sich der Kolbenteil (250) für das Einstechen vorwärts bewegt, ein Teil des Kolbenteils (250) die Seitenfläche des anisotrop-geformten Abschnitts trifft, so dass eine weitere Vorwärtsbewegung des Kolbenteils (250) verhindert wird, und
wobei die Seitenfläche, welche der Teil des Kolbenteils trifft, zu den anderen Seitenflächen mit den unterschiedlichen Abstandsentfernungen "L" gewechselt werden kann, durch eine derartige Drehung des Scheibenteils, dass der anisotropgeformte Abschnitt um die Rotationsachse von ihm gedreht wird, und dadurch der Abstand, über welchen sich der Kolbenteil für das Einstechen vorwärts bewegen kann, geändert wird.

6. Injektor nach Anspruch 5, wobei der Achsenteil (296) flexibel ist.

7. Injektor nach Anspruch 6, wobei der Achsenteil (296) Flexibilität in der Einstechrichtung und einer Rückwärtsrichtung davon aufweist.

8. Injektor nach einem der Ansprüche 5 bis 7, wobei der Achsenteil und der Scheibenteil als ein integraler Teil vorgesehen sind, wobei der Achsenteil und der Scheibenteil miteinander integriert sind.

## Revendications

1. Injecteur pour lancer une lancette afin de fournir une ponction, l'injecteur comprenant:
une partie de piston plongeur (250) pouvant lancer la lancette (100) dans une direction de ponction ; et
un boîtier d'injecteur (270), la partie de piston plongeur (250) étant positionnée dans un espace intérieur du boîtier (270),
dans lequel la partie de piston plongeur (250) comprend une partie de fixation de lancette (251) dans sa pointe,
**caractérisé en ce que**
la partie de fixation de lancette (251) a une double structure cylindrique composée d'un cylindre externe (252) et d'un cylindre interne (253) de sorte que des corps respectifs des cylindres externe (252) et interne (253) sont espacés l'un de l'autre,
dans lequel, lorsque la lancette (100) est fixée à la partie de fixation de lancette (251), le cylindre interne (253) de la partie de fixation de lancette (251) est déplacé pour s'expanser vers l'extérieur, alors que le cylindre externe (252) de la partie de fixation de lancette n'est pas déplacé.

2. Injecteur selon la revendication 1, dans lequel la partie de fixation de lancette (251) de la partie de piston plongeur (250) est positionnée à l'intérieur de la partie d'ouverture d'extrémité avant du boîtier d'injecteur (270), et
dans lequel la surface de corps externe du cylindre externe de la partie de fixation de lancette (251) et une surface de paroi interne de la partie d'ouverture d'extrémité avant du boîtier d'injecteur (270) sont en contact immédiat entre elles ou adjacentes entre elles.

3. Injecteur selon l'une quelconque des revendications 1 à 2, comprenant en outre:
une partie de ressort « A » (282) pour lancer la partie de piston plongeur (250) dans la direction de ponction; et
une partie de ressort « B » (284) pour ramener la partie de piston plongeur (250) lancée,
dans lequel les parties de ressort « A » et « B » sont positionnées parallèlement entre elles dans une direction perpendiculaire à la direction de ponction.

4. Injecteur selon la revendication 3, dans lequel une partie latérale d'extrémité arrière de la partie de piston plongeur (250) est prévue avec deux parties creuses « A » et « B », les parties creuses « A » et « B » étant adjacentes en étant parallèles entre elles,
dans lequel la partie de ressort « A » est positionnée dans la partie creuse « A » de la partie de piston plongeur, alors que la partie de ressort « B » est positionnée dans la partie creuse « B » de la partie de piston plongeur.

5. Injecteur selon l'une quelconque des revendications 1 à 4, comprenant en outre un mécanisme d'ajustement de profondeur de ponction, le mécanisme comprenant:
une partie de disque (294) prévue avec une partie formée par voie anisotrope ayant des faces latérales (295a/b/c/d) et un axe de rotation, des distances espacées « L » entre les faces latérales respectives et l'axe de rotation étant différentes les unes des autres dans la partie formée par voie anisotrope; et
une partie d'axe (296) s'étendant à travers l'axe de rotation de la partie formée par voie anisotrope,
dans lequel la partie de disque (294) est positionnée de sorte qu'au moins l'une des faces latérales de la partie formée par voie anisotrope prend une forme d'une face perpendiculaire par rapport à la direction de ponction,
dans lequel la partie d'axe (296) s'étend dans une direction transversale de l'injecteur,
dans lequel, lorsque la partie de piston plongeur (250) se déplace vers l'avant pour la ponction, une partie de la partie de piston plongeur (250) heurte la face latérale de la partie formée par voie anisotrope de sorte qu'un autre mouvement vers l'avant de la partie de piston plongeur (250) est empêché, et
dans lequel la face latérale que la partie de la partie de piston plongeur heurte, peut être commutée aux autres faces latérales avec des distances différentes espacées « L » par une rotation telle de la partie de disque que la partie formée par voie anisotrope tourne autour de son axe de rotation, et ainsi la distance sur laquelle la partie de piston plongeur peut se déplacer vers l'avant pour la ponction, est modifiée.

6. Injecteur selon la revendication 5, dans lequel la partie d'axe (296) est flexible.

7. Injecteur selon la revendication 6, dans lequel la partie d'axe (296) laisse apparaître la flexibilité dans la direction de ponction et sa direction inverse.

8. Injecteur selon l'une quelconque des revendications 5 à 7, dans lequel la partie d'axe et la partie de disque sont prévues comme étant une partie intégrale, la partie d'axe et la partie de disque étant intégrées l'une par rapport à l'autre.
